# EUROPEAN PATENT APPLICATION

(11) **EP 4 609 880 A1**
(43) Date of publication of application: **03.09.2025**
(21) Application number: 23881965.0
(22) Date of filing: 27.10.2023
(51) Int. Cl.: A61K 47/68, C07K 16/28, C12N 15/13, A61K 39/395, A61K 31/704, A61P 35/00

(54) **PREPARATION CONTAINING ANTI-NECTIN-4 ANTIBODY DRUG CONJUGATE AND USE THEREOF**

(30) Priority: 28.10.2022 CN 202211341423
(71) Applicant: Jiangsu Mabwell Health Pharmaceutical R&D Co., Ltd., China Medical City Taizhou, Jiangsu 225300 (CN); Mabwell (Shanghai) Bioscience Co., Ltd., Shanghai 201210 (CN)
(72) Inventor: CHEN, Kun, Taizhou, Jiangsu 225300 (CN); WU, Lingli, Taizhou, Jiangsu 225300 (CN); JI, Rongyu, Taizhou, Jiangsu 225300 (CN); MEI, Fei, Taizhou, Jiangsu 225300 (CN); TAN, Xiaoding, Taizhou, Jiangsu 225300 (CN)
(74) Representative: Schiweck Weinzierl Koch Patentanwälte Partnerschaft mbB
(86) International application number: PCT/CN2023/127170
(87) International publication number: WO 2024/088390

(57) **Abstract**

The present invention provides a preparation containing an anti-Nectin-4 Antibody-drug conjugate (ADC) and a use thereof. The preparation comprises an ADC, a buffering agent and an excipient. The anti-Nectin-4 ADC in the preparation exists stably, proteins do not aggregate after long-term storage, small molecule drugs are not prone to becoming detached, the osmotic pressure of the preparation is close to isotonic, and targeted therapy of Nectin-4 related diseases is facilitated.

## Description

### CROSS-REFERENCE TO RELATED APPLICATION(S)

The present patent application claims the benefit of priority to Chinese Patent Application No. CN202211341423.0 filed on 28 October 2022, which is incorporated herein by reference in its entirety for all purposes.

### TECHNICAL FIELD

The invention belongs to the field of biological formulations, and particularly the invention relates to a formulation comprising an antibody-drug conjugate.

### BACKGROUND OF THE INVENTION

Antibody-drug conjugate (ADC) is a novel therapeutic for tumor treatment, which is generally composed of an antibody or antibody-like ligand, a small molecule drug, and a linker coupling the two together. Antibody-drug conjugate (ADC) combines the anti-tumor activity of the small molecule drug with the high selectivity of the antibody or antibody-like ligand, and currently is an attentive hotspot in the field of tumor treatment.

Nectins (poliovirus receptor-like molecules) are a novel class of cell adhesion proteins, which regulate cell-cell adhesions either cooperatively with or independently of cadherins. Nectins comprises a family of four members, Nectin-1, -2, -3, and -4, all of them have one extracellular region with three Ig-like loops, one transmembrane segment and one cytoplasmic tail. Among those members, Nectin-4 is specifically expressed in embryo and placenta as well as tumor cells, and has been found in some studies to be closely related to the generation and development of a variety of tumor cells. For example, an analysis on pathological sections derived from 2394 tumor patients revealed that Nectin-4 was widely expressed in the patient populations suffering from bladder cancer, breast cancer and pancreatic cancer. Therefore, Nectin-4 has become an important target for the diagnosis and treatment of many tumors or cancers.

Anti-Nectin-4 antibody-drug conjugates have been prepared and are formed by chemically crosslinking anti-Nectin-4 antibodies to small molecule drugs via linkers. By leveraging the antibodies' targeting specificity toward Nectin-4-expressing cells and potent internalization mediated through binding to targeted antigens, combined with small molecule drugs' effects, those ADCs achieve superior tumor-killing efficacy.

ADCs exhibit poor stability in liquid formulations, for example, exhibiting drug shedding and increased aggregate formation during storage, which is why they are typically formulated as lyophilized formulations preferentially. However, both liquid and lyophilized formulations are required to provide a stabilizing formulation composition to maintain the stability of the contained ADCs.

### SUMMARY OF THE INVENTION

In order to solve the technical problem as above, the present disclosure provides a formulation of defined composition comprising an anti-Nectin-4 antibody-drug conjugate.

The present disclosure provides technical solutions as follows.

In one aspect, the present disclosure provides a liquid formulation comprising an anti-Nectin-4 antibody-drug conjugate or salt thereof as well as a buffer and an auxiliary material.

In the liquid formulation provided by the present disclosure, the antibody-drug conjugate or salt thereof can be prepared according to the method described in Chinese patent application CN202210475286.3, and structural proof and characterization of properties are also detailed in Chinese patent application CN202210475286.3.

In particular, the anti-Nectin-4 antibody-drug conjugate or salt thereof has a formula: Ab-[L-CTD]m, in which Ab represents the anti-Nectin-4 antibody or fragment thereof, L represents a linker, CTD represents a drug, and m represents the average number of drug molecules conjugated to one Ab molecule (average DAR).

Preferably, in the antibody-drug conjugate or salt thereof, CTD is a cytotoxic drug; preferably, CTD is one or more selected from the group consisting of: microtubule inhibitors MMAE, DM1, DM4, Tublysin, amanitin, cachimycin, Eribulin and derivatives thereof; topoisomerase inhibitors SN38, Exatecan and derivatives thereof; and DNA binding agents PBD, doxorubicin and derivatives thereof.

m is 1.0 to 5.0, preferably 3.0 to 4.2, more preferably 3.5 to 4.5, still more preferably 3.8 to 4.2, yet more preferably 3.9 to 4.1, and particularly preferably 4.0.

In the liquid formulation provided by the present disclosure, the antibody-drug conjugate or salt thereof has a structure represented by the formula I as follows: 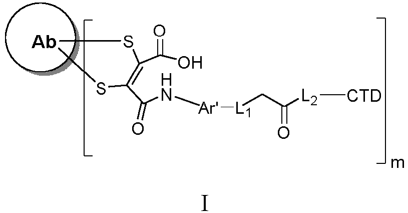 in which:
Ab is the anti-Nectin-4 antibody or fragment thereof;
Ar' is any one selected from the group consisting of: substituted or unsubstituted C6-C10 arylene and substituted or unsubstituted 5-12 membered heteroarylene, wherein the substitution refers to the replacement of a hydrogen atom on a group by one or more substituents selected from the group consisting of: halogen (F, Cl, Br or I), halogenated alkyl (e.g. halogenated C1-C6 alkyl, preferably halogenated C1-C4 alkyl, e.g. trifluoromethyl) and alkoxy (e.g. C1-C6 alkoxy, preferably C1-C4 alkoxy, e.g. methoxy);
L₁ is -O(CH2CH2O)n- linked to Ar', in which n is any integer in the range from 1 to 24, preferably from 1 to 10, more preferably from 3 to 5;
L₂ is an enzyme cleavable fragment, e.g. a dipeptide or a tripeptide or a tetrapeptide or a combination of the dipeptide or the tripeptide or the tetrapeptide with a self-immolative linker (i.e., a polypeptide fragment consisting of 2-4 amino acids, or a combination of the polypeptide fragment with a self-immolative linker), such as Val-Ala, Val-Ala-PAB, Val-Cit, Val-Cit-PAB, Phe-Lys-PAB, Ala-Ala-Ala, Gly-Gly-Phe-Gly (GGFG), MAC glucuronide phenol.

Preferably, L₂-CTD is VcMMAE, GGFG-Dxd or VC-seco-DUBA.

Preferably, in the case that Ar' is a substituted or unsubstituted 5-12 membered heteroarylene, the heteroatom is N.

Preferably, Ar' is a substituted or unsubstituted C6 arylene or a substituted or unsubstituted 6 membered heteroarylene.

According to particular embodiments of the invention, the anti-Nectin-4 antibody-drug conjugate or salt thereof has a structure as follows:

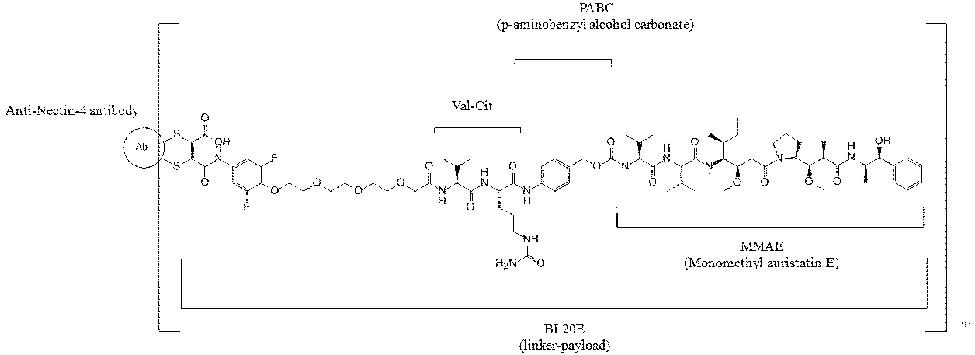

Preferably, the ADC having the structure shown above has an average DAR of 3.8-4.2, yet preferably 3.9-4.1, particularly preferably 4.0.

In the liquid formulation according to the present invention, the anti-Nectin-4 antibody drug conjugate or salt thereof comprises an anti-Nectin-4 antibody or fragment thereof, i.e., the "Ab" in the structure shown above. The fragment of the antibody encompasses various functional fragments of the antibody, for example, an antigen-binding portions thereof, such as an Fab, F(ab')2, or scFv fragment.

In particular, the anti-Nectin-4 antibody or fragment thereof comprises three heavy chain complementarity determining regions, i.e., H-CDR1, H-CDR2, and H-CDR3, and three light chain complementarity determining regions, i.e., L-CDR1, L-CDR2, and L-CDR3, wherein:
the H-CDR1 comprises the amino acid sequence (DYGVS) shown in SEQ ID NO. 3, the H-CDR2 comprises the amino acid sequence (VIWGGGKIYYNSVLKS) shown in SEQ ID NO. 4, and the H-CDR3 comprises the amino acid sequence (QGGLLFYAMDY) shown in SEQ ID NO. 5; and, the L-CDR1 comprises the amino acid sequence (KSSQSLLNTYSQKNYLA) shown in SEQ ID NO. 8, the L-CDR2 comprises the amino acid sequence (FASTRES) shown in SEQ ID NO. 9, and the L-CDR3 comprises the amino acid sequence (QQHYNTPFT) shown in SEQ ID NO. 10.

Preferably, the anti-Nectin-4 antibody or fragment thereof comprises a heavy chain variable region (VH) comprising the amino acid sequence shown in SEQ ID NO: 2 or a variant thereof; and, the anti-Nectin-4 antibody or fragment thereof comprises a light chain variable region (VL) comprising the amino acid sequence shown in SEQ ID NO: 7 or a variant thereof.

In the context of the present disclosure, a "variant" of an amino acid sequence refers to an amino acid sequence having at least 75% sequence identity (any percent identity greater than or equal to 75%, e.g., at least 80%, preferably at least 85%, more preferably at least 90%, further preferably at least 91%, 92%, 93%, 94%, 95%, 96%, 97%, 98%, or even 99% identity, etc.) to the amino acid sequence.

In particular, the anti-Nectin-4 antibody or fragment thereof defined in the present disclosure comprises at least a heavy chain variable region and a light chain variable region, both comprising CDRs as above and interspersed Framework Regions (FRs), which domains are arranged as follows: FR1-CDR1-FR2-CDR2-FR3-CDR3-FR4. Thus, with respect to the heavy chain variable region and the light chain variable region comprised in the anti-Nectin-4 antibody or fragment thereof provided by the present disclosure, the up to 25% difference in the amino acid sequence resulted from the "at least 75% sequence identity" may be present in any framework region in the heavy chain variable region or the light chain variable region. Or, with respect to the anti-Nectin-4 antibody or fragment thereof provided by the present disclosure as a whole, the up to 25% difference may be present in any domain or sequence in the antibody or fragment thereof defined in the present disclosure other than the heavy chain variable region and the light chain variable region. The difference may be resulted from amino acid deletion, addition or substitution at any position, and the substitution may be conservative substitution or non-conservative substitution.

**In** the antibody-drug conjugate or salt thereof defined in the present disclosure, the anti-Nectin-4 antibody or fragment thereof is in any form, e.g., a monoclonal antibody, a single chain antibody, a bispecific antibody, a single domain antibody, a nanobody, a fully or partially humanized antibody, or a chimeric antibody and the like against Nectin-4; alternatively, the antibody or fragment thereof is a half-antibody or an antigen-binding fragment of the half-antibody against Nectin-4, e.g., single-chain variable fragment (scFv), bivalent single-chain variable fragment (BsFv), disulfide-stabilized variable fragment (dsFv), (disulfide-stabilized variable fragment)2 ((dsFv)2), antigen-binding fragment (Fab), Fab' fragment (Fab'), F(ab')2 fragment (F(ab')2), or variable fragment (Fv). With respect to the fragment of the antibody provided by the present disclosure, preferably, the fragment is any fragment of the antibody capable of binding to Nectin-4. Further, the Nectin-4 is mammal Nectin-4, preferably primate Nectin-4, more preferably human Nectin-4.

Preferably, in the antibody-drug conjugate or salt thereof defined in the present disclosure, the anti-Nectin-4 antibody or fragment thereof may further comprise a constant region. Preferably, the anti-Nectin-4 antibody or fragment thereof further comprises a human or murine heavy chain constant region (CH) and/or light chain constant region (CL), more preferably a heavy chain constant region selected from the group consisting of heavy chain constant regions of IgG, IgA, IgM, IgD and IgE and/or a kappa or lambda type light chain constant region.

Preferably, the antibody is a monoclonal antibody, preferably a murine, chimeric, or humanized monoclonal antibody; more preferably, the heavy chain constant region of the monoclonal antibody is of IgG1 or IgG4 subtype and the light chain constant region of the monoclonal antibody is of kappa type. Alternatively, for example, the antibody is an immunoglobulin, in particular IgA, IgD, IgE, IgG or IgM, e.g., a human subtype of IgA, IgD, IgE, IgG or IgM, more preferably a human IgG1, IgG2, IgG3 or IgG4 subtype.

According to particular embodiments of the invention, the anti-Nectin-4 antibody or fragment thereof comprised by the antibody-drug conjugate or salt thereof comprises a heavy chain (HC) comprising the amino acid sequence shown in SEQ ID NO: 1 or a variant thereof; and, a light chain (LC) comprising the amino acid sequence shown in SEQ ID NO: 6 or a variant thereof.

In the context of the present disclosure, the "salt" of the anti-Nectin-4 antibody-drug conjugate refers to a pharmaceutically acceptable salt thereof, for example, an inorganic salt or an organic salt, e.g. a sodium salt.

The formulation according to the invention may be a liquid formulation, for example, in the form of a solution, an emulsion or a suspension, e.g., in water and/or in a buffer. Preferably, the liquid formulation is a liquid formulation for parenteral administration. Parenteral administration may be intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, intrasternal administration. Preferably, the liquid formulation is a liquid composition for intravenous administration, such as for intravenous drip.

**In** the liquid formulation according to the invention, the buffer is one or more selected from the group consisting of phosphate buffer, acetate buffer, and Tris-HCl buffer. Preferably, the phosphate buffer comprises a mixture of sodium dihydrogen phosphate and disodium hydrogen phosphate. Preferably, the acetate buffer comprises a mixture of acetic acid and sodium acetate.

The liquid formulation according to the invention further comprises an auxiliary material, which may function as an excipient, an osmolality modifier, a protein stabilizer, a solubilizing agent, or the like.

Preferably, the auxiliary material comprises a surfactant. Preferably, the surfactant is polysorbate 20 or polysorbate 80.

Preferably, the auxiliary material comprises a sugar, preferably trehalose. Optionally, the auxiliary material comprises arginine.

The liquid formulation according to the invention has a pH of 5.0-8.0, preferably 7.0-8.0, more preferably 7.0-7.8, such as 7.2, 7.4, 7.6, or 7.8.

Preferably, the liquid formulation according to the invention comprises the anti-Nectin-4 antibody-drug conjugate or salt thereof in a concentration of 5-40 mg/mL, preferably 8-30 mg/mL, more preferably 10-20 mg/mL (e.g, 10, 15, or 20 mg/mL).

Preferably, the liquid formulation according to the invention comprises the phosphate buffer acting as a buffering system. Preferably, the liquid formulation comprises the phosphate buffer in a concentration of 5-20 mmol/L, preferably 8-15 mmol/L, more preferably 8-10 mmol/L.

Preferably, the liquid formulation according to the invention comprises trehalose. Preferably, the liquid formulation comprises trehalose in a concentration of 5%-9% (w/v), preferably 6%-8% (w/v), more preferably 7% (w/v).

Preferably, the liquid formulation according to the invention comprises polysorbate 20 or polysorbate 80 acting as a surfactant, such as polysorbate 20 or polysorbate 80 in a concentration of 0.0003%-0.3% (w/v). Preferably, the liquid formulation comprises polysorbate 20 in a concentration of 0.0003%-0.3% (w/v), preferably 0.01%-0.03% (w/v), more preferably 0.02%-0.03% (w/v).

In the present disclosure, "w/v" refers to weight (mg) per volume (L).

Further preferably, the present disclosure provides a formulation, such as a liquid formulation, comprising:
5-40 mg/mL the anti-Nectin-4 antibody-drug conjugate or salt thereof;
5-20 mmol/L the phosphate buffer;
5%-9% (w/v) the sugar, such as trehalose; as well as
0.0003%-0.3% (w/v) the surfactant, such as polysorbate 20;
and, the liquid formulation has a pH of 5.0-8.0.

Further preferably, the present disclosure provides a formulation, such as a liquid formulation, comprising:
8-30 mg/mL the anti-Nectin-4 antibody-drug conjugate or salt thereof;
5-20 mmol/L the phosphate buffer;
6%-9% (w/v) the sugar, such as trehalose; as well as
0.01%-0.03% (w/v) the surfactant, such as polysorbate 20;
and, the liquid formulation has a pH of 7.0-8.0, such as 7.2-7.8.

Further preferably, the present disclosure provides a formulation, such as a liquid formulation, comprising:
10-30 mg/mL the anti-Nectin-4 antibody-drug conjugate or salt thereof;
8-15 mmol/L the phosphate buffer;
6%-8% (w/v) the sugar, such as trehalose; as well as
0.02%-0.03% (w/v) the surfactant, such as polysorbate 20;
and, the liquid formulation has a pH of 7.2-7.8.

Further preferably, the present disclosure provides a formulation, such as a liquid formulation, comprising:
10-20 mg/mL the anti-Nectin-4 antibody-drug conjugate or salt thereof;
8-10 mmol/L the phosphate buffer;
6%-8% (w/v) the sugar, such as trehalose; as well as
0.02%-0.03% (w/v) the surfactant, such as polysorbate 20;
and, the liquid formulation has a pH of 7.2-7.6.

According to particular embodiments of the invention, the present disclosure provides a formulation, such as a liquid formulation, comprising:
10 mg/mL the anti-Nectin-4 antibody-drug conjugate or salt thereof;
10 mmol/L the phosphate buffer;
7% trehalose; as well as
0.02% polysorbate 20;
and, the liquid formulation has a pH of 7.4.

The formulation according to the invention may be a solid formulation obtained by solidifying the liquid formulation provided by the present disclosure. Preferably, the solid formulation is a lyophilized formulation, such as lyophilized powder injection.

According to particular embodiments of the invention, the solid formulation is a lyophilized formulation which has a moisture content of no greater than 3.0%, preferably of no greater than 1.5%, more preferably of no greater than 1.0%.

Upon reconstitution of the lyophilized formulation provided by the present disclosure in a solvent (e.g., a pharmaceutically acceptable solvent, such as water for injection), the formulation obtained comprises:
the anti-Nectin-4 antibody-drug conjugate or a salt thereof in a concentration of 5-40 mg/mL, preferably 8-30 mg/mL, more preferably 10-20 mg/mL (e.g., 10, 15, or 20 mg/mL);
the phosphate buffer in a concentration of 5-20 mmol/L, preferably 8-15 mmol/L, more preferably 8-10 mmol/L;
trehalose in a concentration of 5%-9% (w/v), preferably 6%-8% (w/v), more preferably 7% (w/v); and/or
polysorbate 20 in a concentration of 0.0003%-0.3% (w/v), preferably 0.01%-0.03% (w/v), more preferably 0.02%-0.03% (w/v).

Upon reconstitution of the lyophilized formulation provided by the present disclosure in a pharmaceutically acceptable solvent, such as water for injection, the formulation obtained has a pH of 5.0-8.0, preferably 7.0-8.0, more preferably 7.0-7.8, such as 7.2, 7.4, 7.6, or 7.8.

Alternatively, upon reconstitution of the lyophilized formulation provided by the present disclosure in a solvent (e.g., a pharmaceutically acceptable solvent, such as water for injection), a liquid formulation according to the invention is obtained.

The lyophilized formulation provided by the present disclosure can be obtained by lyophilizing the liquid formulation according to the invention. Preferably, the lyophilized formulation is prepared by the method including the steps as follows:
(1) pre-freezing the liquid formulation at ≤ -40 °C for at least 2 hours;
(2) raising the temperature of the pre-frozen product obtained to -35 °C to -20 °C, and annealing for at least 2 hours;
(3) lowering the temperature of the annealed product obtained to ≤ -40 °C, and pre-freezing again for at least 2 hours;
(4) raising the temperature of the pre-frozen product obtained to -20 ± 10 °C and drying under a pressure of ≤ 0.2 mbar for at least 24 hours;
(5) raising the temperature of the dried product obtained to 20 ± 5 °C and drying under a pressure of ≤ 0.2 mbar for at least 10 hours.

Preferably, the method is performed in a freeze-dryer, and the temperatures specified refer to the shelf temperature of the freeze-dryer.

The formulation according to the invention may also be a reconstituted formulation obtained by dissolving the solid formulation provided by the present disclosure in a solvent (a pharmaceutically acceptable solvent, such as water for injection). Preferably, the pharmaceutically acceptable solvent is water (e.g., water for injection) or an isotonic solution (e.g., 0.9% NaCl solution and 5% glucose solution).

**In** another aspect, the present disclosure provides use of the formulation according to the invention (including the liquid formulation, the solid formulation and the reconstituted formulation) in the manufacture of a medicament for treating a tumor. The medicament may be used for parenteral administration of the formulation, or for parenteral administration of a formulation obtained by further formulating the formulation. Parenteral administration may be intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, intrasternal administration. Preferably, the medicament is used for intravenous administration (such as for intravenous drip) of the formulation, or for intravenous administration (such as for intravenous drip) of a formulation obtained by further formulating the formulation.

Alternatively, the present disclosure provides a method for treating a tumor, comprising administering to a subject in need thereof (e.g., a therapeutically effective amount of) the formulation according to the invention (including the liquid formulation, the solid formulation and the reconstituted formulation) or a formulation obtained by further formulating the formulation. That administering may be parenteral administration. Parenteral administration may be intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, intrasternal administration. Preferably, the administering is intravenous administration, such as intravenous drip.

The subject is a mammal, preferably a primate, more preferably a human.

Preferably, the tumor is a tumor or cancer associated with high expression of Nectin-4. More preferably, the tumor or cancer is any one selected from the group consisting of: bladder cancer, breast cancer, ovarian cancer, pancreatic cancer, hepatocellular cancer, gastric cancer, non-hodgkin's lymphoma, hodgkin's lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumor, prostate cancer, colorectal cancer, glioma, and mesothelioma.

The present disclosure also provides a kit comprising the formulation according to the invention (including the liquid formulation, the solid formulation and the reconstituted formulation) or a formulation obtained by further formulating the formulation. The kit may be used for administration of the formulation as above, or for administration of a formulation obtained by further formulating the formulation as above. Optionally, the kit may also include other reagents or means for the administration as described above, such as one or more of water, isotonic solution, syringe, and the like.

Compared to prior arts, the present disclosure provides a formulation comprising a novel anti-Nectin-4 antibody-drug conjugate. The anti-Nectin-4 antibody-drug conjugate in the formulation remains stable with minimal protein aggregation and negligible shedding of the small molecule drugs over long-term storage; and the formulation has an osmotic pressure near to isotonic. These properties collectively enhance the formulation's suitability for targeted therapy of Nectin-4-associated diseases.

### BRIEF DESCRIPTION OF THE DRAWINGS

Embodiments of the invention are described in detail below with reference to the attached figures, in which:
Figure 1A shows the changes in HIC main peaks of the formulations having different pH, detected in the first round screening;
Figure 1B shows the changes in SEC main peaks of the formulations having different pH, detected in the first round screening;
Figure 2A shows the changes in HIC main peaks of the formulations having different pH and different buffers, detected in the second round screening;
Figure 2B shows the changes in HIC main peaks of the formulations having different pH and different ADC concentrations, detected in the second round screening;
Figure 2C shows the changes in SEC main peaks of the formulations having different pH and different ADC concentrations, detected in the second round screening;
Figure 3A shows the changes in HIC main peaks of the formulations having different trehalose concentrations, detected in the third round screening;
Figure 3B shows the changes in HIC main peaks of the formulations having different ADC concentrations and different polysorbates, detected in the third round screening;
Figure 3C shows the changes in SEC main peaks of the formulations having different types and concentrations of auxiliary materials, detected in the third round screening; and
Figure 4 shows the lyophilization curve obtained in the validation of the lyophilization process provided in the present disclosure.

### DETAILED DESCRIPTION OF PREFERRED EMBODIMENTS

The invention is illustrated below with reference to specific examples. It will be understood by those skilled in the art that these examples are merely illustrative of the invention and do not limit the scope of the invention in any way.

Experimental procedures in the following examples are all conventional ones, unless otherwise specified. Raw materials and reagents used in the following examples are all commercially available products, unless otherwise specified.

Following detection methods are used in the present disclosure.

### I. SEC-HPLC detection

High performance liquid chromatograph apparatus: Waters.

Chromatographic column: TOSOH, TSKgel G3000SWXL.

Mobile phase: 100 mmol/L phosphate buffer (PB), 200 mmol/L L-arginine hydrochloride, and 5% isopropanol (pH 6.8).

Sample preparation and analysis: dilute a sample to 1.0 mg/mL with the mobile phase and centrifuge at 12000 rpm for 10 min; and then take the supernatant obtained for analysis.

Analysis conditions:
Flow rate: 0.6 mL/min;
Column temperature: 30 °C;
Detection wavelength: 280 nm.

System suitability test: analyze 20 µL of a working reference standard under the analysis conditions. The monomer peak shall exhibit a theoretical plate number of no less than 5000.

### (II) RP-HPLC detection

High performance liquid chromatograph apparatus: Waters.

Chromatographic column: Waters Acquity UPLC BEH-C18 (1.7 µm, 2.1 mm × 50 mm).

Mobile phase A: 0.1% phosphoric acid aqueous solution.

Mobile phase B: acetonitrile-0.1% phosphoric acid aqueous solution.

Blank diluent: a mixture of DMSO, purified water, and acetonitrile in 1: 1: 2 by volume.

Preparation of standards: use small molecule compounds MMAE, L20E and BL20E as standards for detecting the content of free small molecule drugs.

Accurately weigh 10 mg each of MMAE, L20E and BL20E standards, dissolve and dilute them to 100 mL with acetonitrile, to obtain a mixed stock solution of standards for detecting free small molecule drugs. Transfer 400 µL of the stock solution to 100 µL of mobile phase A, mix, and add 3.5 mL of the blank diluent and mix, to obtain a mixed solution of the MMAE, L20E and BL20E standards each in a concentration of 10 µg/mL for detecting free small molecule drugs. Dilute the solution further with the blank diluent to obtain a mixed solution of the standards each in a concentration of 1 µg/mL for detecting free small molecule drugs.

Dilute the solutions further to different concentrations as shown below for plotting standard curves.

| Concentration used for plotting standard curve (µg/mL) | Standard solution | Volume of the standard solution (µL) | Volume of the blank diluent (µL) |
|---|---|---|---|
| 0.05 | Mixed solution of the standards, 1 µg/mL | 50 | 950 |
| 0.10 | | 100 | 900 |
| 0.20 | | 200 | 800 |
| 0.50 | | 500 | 500 |
| 1.00 | | 1000 | 0 |
| 2.00 | Mixed solution of the standards, 10 µg/mL | 200 | 800 |
| 5.00 | | 500 | 500 |
| 10.00 | | 1000 | 0 |

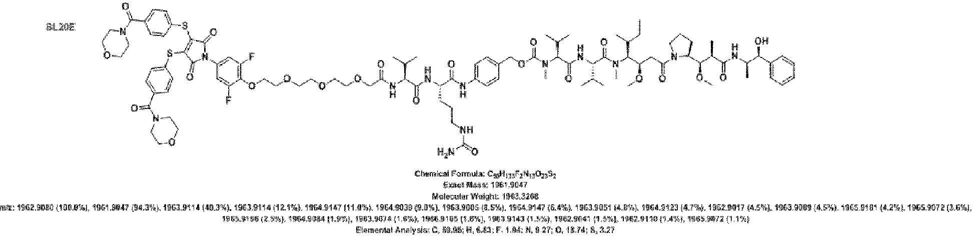

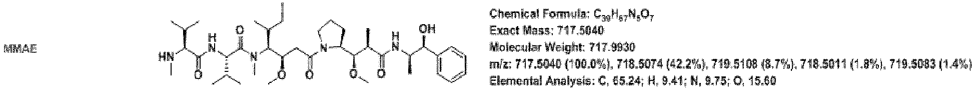

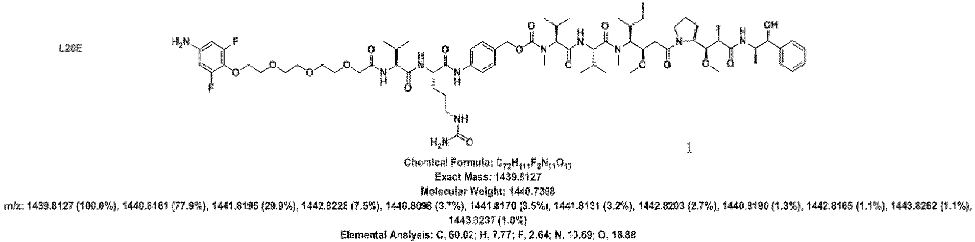

Sample preparation and analysis: add 200 µL of a sample to 800 µL of acetonitrile, mix thoroughly, and centrifuge at 12000 rpm for 10 min; and then take the supernatant obtained for analysis.

Analysis conditions:
Flow rate: 0.5 mL/min;
Column temperature: 60 °C;
Detection wavelength: 210 nm for detecting MMAE; 241 nm for detecting L20E and BL20E. System suitability test: the chromatogram of the blank matrix shall exhibit no chromatographic peaks within the elution window corresponding to any one of the free small molecule drugs. Standard curve linearity: establish standard curves by plotting the concentrations of the standards against peak areas, and calculate regression equations and correlation coefficients. Correlation coefficients (R²) shall be > 0.99.

### (III) HIC-HPLC detection

High performance liquid chromatograph apparatus: Waters.

Chromatographic column: Proteomix HIC Butyl-NP5 (4.6 mm × 35 mm).

Mobile phase A: 0.025 mol/L sodium phosphate, and 1.2 mol/L ammonium sulphate (pH 7.0).

Mobile phase B: 0.025 mol/L sodium phosphate (pH 7.0).

Mobile phase C: 100% IPA.

Sample preparation and analysis: dilute a sample to 1.0 mg/mL with mobile phase B and centrifuge at 12000 rpm for 5 min; and then take the supernatant obtained for analysis.

Analysis conditions:
Flow rate: 0.8 mL/min;
Column temperature: 25 °C;
Detection wavelength: 280 nm.

System suitability test: after equilibration, inject mobile phase B as a blank control sample and run the system. The blank control sample shall exhibit no interfering peaks within the integration window.

### Example 1 Preparation of ADC

An antibody-drug conjugate comprising an anti-Nectin-4 humanized monoclonal antibody was prepared. The anti-Nectin-4 humanized monoclonal antibody is described in Chinese patent application CN202210475286.3, abbreviated as "hH2L1". The heavy and light chains of the antibody as well as variable regions and Complementarity Determining Regions (CDRs) therein are shown below.
> amino acid sequence of the heavy chain H2, with variable regions in italics and underlined and CDRs in bold further (according to the KABAT numbering scheme) 
EVQLQESGPGLVKPSETLSLTCTVSGFSLIDYGVSWIRQPPGKGLEWIGVIWGGGKIYYNSVLK
SRVTISKDNSKSQVSLKLSSVTAADTAVYYCAKQGGLLFYAMDYWGQGTLVTVSSASTKGPSVF
PLAPSSKSTSGGTAALGCLVKDYFPEPVTVSWNSGALTSGVHTFPAVLQSSGLYSLSSVVT
VPSSSLGTQTYICNVNHKPSNTKVDKRVEPKSCDKTHTCPPCPAPELLGGPSVFLFPPKPK
DTLMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSV
MHEALHNHYTQKSLSLSPGK
   HC: SEQ ID NO. 1;
   VH: SEQ ID NO. 2;
   H-CDR1: SEQ ID NO. 3; H-CDR2: SEQ ID NO. 4; H-CDR3: SEQ ID NO. 5
> amino acid sequence of the light chain L1, with variable regions in italics and underlined and CDRs in bold further (according to the KABAT numbering scheme) 
DIVMTQSPDSLAVSLGERATINCKSSQSLLNTYSQKNYLAWYQQKPGQPPKLLIYFASTRESG
VPDRFSGSGSGTDFTLTISSLQAEDVAVYYCQQHYNTPFTFGGGTKVEIKRTVAAPSVFIFPPS
DEQLKSGTASVVCLLNNFYPREAKVQWKVDNALQSGNSQESVTEQDSKDSTYSLSSTLT
LSKADYEKHKVYACEVTHQGLSSPVTKSFNRGEC
   LC: SEQ ID NO. 6;
   VL: SEQ ID NO. 7;
   L-CDR1: SEQ ID NO. 8; L-CDR2: SEQ ID NO. 9; L-CDR3: SEQ ID NO. 10

### (1) Antibody reduction:

The antibody protein was diluted to 10 ± 2 g/L using a 10 mmol/L histidine buffer (pH 6.0 ± 0.1), and then a 10 ± 2 g/L aqueous solution of the reducing agent TCEP (tris(2-carboxyethyl)phosphine) at a molar ratio of 10:1 (TCEP : antibody) was added to reduce the antibody. The antibody was reduced at 25 ± 5 °C for at least 120 minutes. Subsequently the reaction system was concentrated to 15 g/L and then subjected to ultrafiltration exchange with ≥ 7 volumes of a buffer (50 mmol/L PB, 50 mmol/L NaCl, and 2 mmol/L EDTA·Na₂, pH 7.0 ± 0.2).

### (2) Coupling:

8.5% DMA (dimethylacetamide) was added as a pre-solvent into the reaction system obtained from step (1). Next, a DMA solution containing the linker-payload BL20E at a molar ratio of 4.8 ± 0.3 : 1 (linker-payload : antibody) was added, and then the reaction mixture obtained was agitated at 25 ± 5 °C for at least 60 minutes.

### (3) Ultrafiltration:

The reaction system obtained from step (2) was subjected to ultrafiltration exchange with ≥ 7 volumes of a buffer (50 mmol/L PB, pH 7.8 ± 0.2) to remove free small molecule drugs and residual DMA.

### (4) Hydrolysis:

The temperature of the reaction system obtained from step (3) was raised to 35 ± 2 °C, and then reaction system was incubated for at least 120 minutes.

### (5) Hydrophobic chromatography:

Butyl Sepharose 4 FF filler or any other fillers with comparable specifications were used. The conductivity of the hydrolyzed solution obtained from step (4) was adjusted with 50 mmol/L PB and 3 mol/L ammonium sulfate, and upon mixed thoroughly, the solution was filtered. The column packed with the filler was equilibrated with 50 mmol/L PB and 0.45 mol/L ammonium sulfate (pH 7.5 ± 0.2), then the solution was loaded. A linear gradient elution using 50 mmol/L PB (pH 7.5 ± 0.2) from 0% to 80% with a linear elution length of 10-12 CVs, were performed. Samples peaks were collected.

An anti-Nectin-4 antibody-drug conjugate having a structure as follows was obtained:

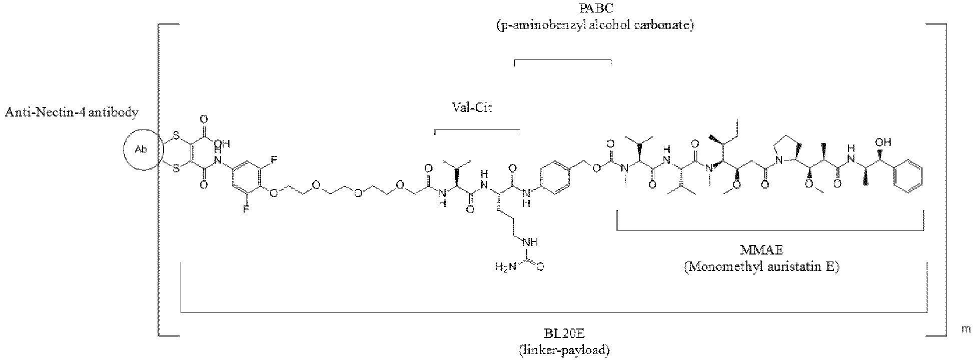

The average DAR of the ADC obtained was measured and the results are shown in Table 1.

**Table 1. Results of DAR values of a sample detected via HIC-HPLC**

| Sample name | Percentage of components having a DAR value of 3 (%) | Percentage of components having a DAR value of 4 (%) | Average DAR |
|---|---|---|---|
| DS | 0.9 | 99.1 | 4.0 |

### Example 2 First round screening (pH) for an ADC formulation

In this example, the accelerated stability of formulations having different pH values was studied, to identify a suitable pH range for a formulation comprising the ADC as prepared in Example 1.

### Study design:

pH range: 5.0 to 8.0;
temperature: 25 ± 2 °C
detection methods: SEC-HPLC and HIC-HPLC.

The pH of a protein formulation is critical for product stability and biological activity. The selection of the pH of a protein formulation is primarily influenced by the physicochemical properties of the protein molecule. At pH values near its isoelectric point (pI), the protein molecule exhibits a tendency to precipitate. Solution pH near the pI may induce protein precipitation, while pH above the pI provides strong alkalinity which is unsuitable for protein preservation. Thus, pH conditions below the pI necessitate evaluation; however, excessive acidity compromises protein stability and may induces small molecule drug shedding.

Testing revealed that the ADC provided by the present disclosure had an isoelectric point of 8.0. Consequently, pH screening was conducted within the range of 5.0 to 8.0. Following ICH stability testing guidelines, 25±2°C was selected as the temperature for this accelerated stability study.

The formulations examined are shown in Table 2.

**Table 2. Experiment design of pH screening**

| Number of the formulation | Buffer | Concentration of the ADC | pH |
|---|---|---|---|
| A | 10 mmol/L acetate | 10 mg/mL | 5.0 |
| B | 10 mmol/L acetate | | 5.5 |
| C | 10 mmol/L PB | | 6.0 |
| D | 10 mmol/L PB | | 6.5 |
| E | 10 mmol/L PB | | 7.0 |
| F | 10 mmol/L PB | | 7.5 |
| G | 10 mmol/L PB | | 8.0 |

| | | | |
|---|---|---|---|
| Note: PB is sodium dihydrogen phosphate-disodium hydrogen phosphate buffer. | | | |

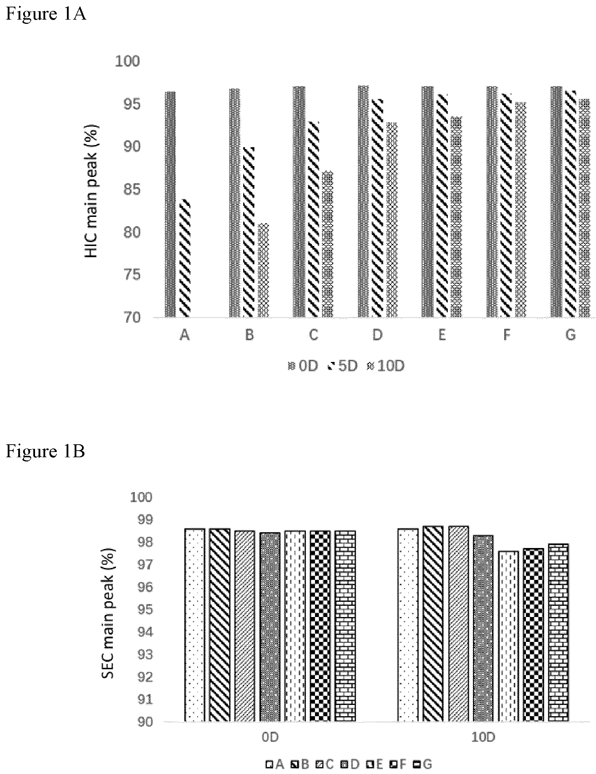

Samples were taken at different time points and detected. The experiment results demonstrated that: during the 10-day accelerated study, 1) The changes in the area percentage of HIC main peaks decreased with increasing pH (shown in Figure 1A & Table 3), indicating decreased small molecule drug shedding and enhanced formulation stability; 2) Purity by SEC decreased with increasing pH (shown in Figure 1B & Table 3), though the changes were minor and remained within limits of acceptability when the pH was in the range of 7.0-8.0.

When developing an antibody-drug conjugate formulation, a pH-dependent stability of both constituents (the macromolecular protein and small molecule drug) needs to be considered. Consequently, the pH screening results above determined the next second round screening to be performed in pH 7.0-8.0.

**Table 3. Experiment results of pH screening**

| Number of the formulation | Buffer | pH | Main peak (SEC) (%) | | | Main peak (HIC) * (%) | | |
|---|---|---|---|---|---|---|---|---|
| | | | 0 d | 5 d | 10 d | 0 d | 5 d | 10 d |
| A | acetate | 5.0 | 98.6 | 99.3 | 98.6 | 96.46 | 83.87 | 70.05 |
| B | acetate | 5.5 | 98.6 | 98.7 | 98.7 | 96.84 | 89.98 | 81.05 |
| C | PB | 6.0 | 98.5 | 98.3 | 98.7 | 97.07 | 92.98 | 87.24 |
| D | PB | 6.5 | 98.4 | 99.2 | 98.3 | 97.15 | 95.59 | 92.86 |
| E | PB | 7.0 | 98.5 | 98.2 | 97.6 | 97.09 | 96.15 | 93.54 |
| F | PB | 7.5 | 98.5 | 98.2 | 97.7 | 97.08 | 96.22 | 95.26 |
| G | PB | 8.0 | 98.5 | 98.8 | 97.9 | 97.12 | 96.57 | 95.60 |

| | | | | | | | | |
|---|---|---|---|---|---|---|---|---|
| *: profiling the small molecule drug shedding (the higher the main peaks, the better the stability of the small molecule drugs). | | | | | | | | |

### Example 3 Second round screening (ADC concentration, buffer and pH) for an ADC formulation

In this example, the accelerated stability of formulations having different ADC concentrations, buffers and pH values was studied, to identify suitable concentration, buffer, and pH range for a formulation comprising the ADC as prepared in Example 1.

### Study design:

pH range: 7.0 to 8.0;
ADC concentrations: 10-20 mg/mL;
buffers: Tris and PB;
temperature: 25 ± 2 °C
detection methods: SEC-HPLC, HIC-HPLC, and RP-HPLC.

In Example 2, a pH range of 7.0-8.0 was identified. Tris and phosphate buffer (PB) systems match that pH range. In view of the pH buffering capacity and buffering range of the two systems, the pH screening range was refined to 7.0-7.8; concurrently, in order to investigate the relationship between the ADC concentration and the product quality, the range of ADC concentrations was set to 10-20 mg/mL, a concentration range which can satisfy the dosage requirements for clinical use in the future while preventing excessive administration volumes.

The formulations examined are shown in Table 4.

**Table 4. Experiment design of ADC concentration, buffer and pH screening**

| Number of the formulation | ADC concentration (mg/mL) | Buffer | pH |
|---|---|---|---|
| A | 20 | 10 mmol/L Tris | 7.0 |
| B | 20 | 10 mmol/L Tris | 7.4 |
| C | 20 | 10 mmol/L Tris | 7.8 |
| D | 10 | 10 mmol/L Tris | 7.0 |
| E | 10 | 10 mmol/L Tris | 7.4 |
| F | 10 | 10 mmol/L Tris | 7.8 |
| G | 20 | 10 mmol/L PB | 7.0 |
| H | 20 | 10 mmol/L PB | 7.4 |
| I | 20 | 10 mmol/L PB | 7.8 |
| J | 10 | 10 mmol/L PB | 7.0 |
| K | 10 | 10 mmol/L PB | 7.4 |
| L | 10 | 10 mmol/L PB | 7.8 |

| | | | |
|---|---|---|---|
| Note: Tris is tris(hydroxymethyl) aminomethane | | | |

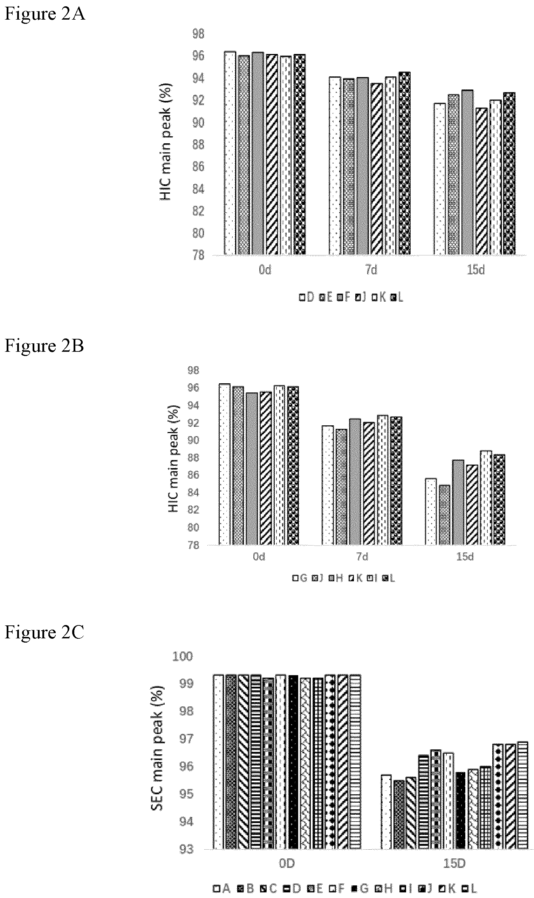

The experiment results demonstrated that: 1) In the PB and Tris buffering systems, the changes in the area percentage of HIC main peaks decreased with increasing pH, and the shedding rates of the small molecule drugs decreased gradually as well; and minimal differences were observed between pH 7.4 and 7.8 (shown in Figure 2A & Table 5). However, pH 7.8 nears to the ADC's pI (8.0), and risks protein precipitation. Consequently, pH 7.4 was selected for the next stage of study; 2) The PB and Tris buffering systems exhibited little difference; however, the pH of Tris buffers is more temperature-sensitive, so PB was selected as the buffer in the next stage of study; 3) When the ADC concentrations were in the range of 10-20 mg/mL, the changes in the shedding rates of the small molecule drugs detected by RP-HPLC were small, while the area percentages of SEC main peaks showed a decreasing tendency with increasing ADC concentrations (shown in Figure 2B and Figure 2C & Table 5).

Based on a comprehensive analysis on the results above, a good formulation comprising PB (pH 7.4) and an ADC concentration of 10 mg/mL was identified and used as a basis for the following third round screening.

**Table 5. Experiment results of ADC concentration, buffer and pH screening**

| Number of the formulation | Buffer | ADC concentration (mg/mL) | Main peak (SEC) (%) | | | Shedding rate of the small molecule drugs (RP) (%) | | |
|---|---|---|---|---|---|---|---|---|
| | | | 0 d | 7 d | 15 d | 0 d | 7 d | 15 d |
| A | Tris pH7.0 | 20 | 99.3 | 97.5 | 95.7 | ND | 0.26 | 1.45 |
| B | Tris pH 7.4 | 20 | 99.3 | 97.4 | 95.5 | ND | 0.24 | 0.96 |
| C | Tris pH 7.8 | 20 | 99.3 | 97.3 | 95.6 | ND | 0.06 | 0.89 |
| D | Tris pH 7.0 | 10 | 99.3 | 97.7 | 96.4 | ND | 0.23 | 0.60 |
| E | Tris pH 7.4 | 10 | 99.2 | 97.8 | 96.6 | ND | 0.23 | 0.51 |
| F | Tris pH 7.8 | 10 | 99.3 | 97.7 | 96.5 | ND | 0.13 | 0.54 |
| G | PB pH 7.0 | 20 | 99.3 | 97.3 | 95.8 | ND | 0.32 | 0.96 |
| H | PB pH 7.4 | 20 | 99.2 | 93.4 | 95.9 | ND | 0.28 | 0.83 |
| I | PB pH 7.8 | 20 | 99.2 | 97.4 | 96.0 | ND | 0.18 | 0.51 |
| J | PB pH 7.0 | 10 | 99.3 | 97.9 | 96.8 | ND | 0.45 | 1.30 |
| K | PB pH 7.4 | 10 | 99.3 | 97.8 | 96.8 | ND | 0.20 | 0.70 |
| L | PB pH 7.8 | 10 | 99.3 | 98.0 | 96.9 | ND | 0.13 | 0.43 |

| | | | | | | | | |
|---|---|---|---|---|---|---|---|---|
| Note: "ND" means no small molecule drug shedding was detected. | | | | | | | | |

### Example 4 Third round screening (auxiliary materials) for an ADC formulation

In this example, the accelerated stability of formulations comprising different types and amounts of auxiliary materials was studied, to identify suitable auxiliary materials, including protective agents and surfactants, for a formulation comprising the ADC as prepared in Example 1.

### Study design:

ADC concentrations: 10-20 mg/mL;
trehalose concentrations: 5% to 9%;
Arginine concentrations: 1% to 5%;
polysorbate 20 concentrations: 0.01% to 0.03%;
temperature: 25 ± 2 °C;
detection methods: SEC-HPLC, HIC-HPLC, and RP-HPLC.

Trehalose is commonly used excipient and stabilizer in injectable formulations, for osmolality adjustment as well as protein protection and stabilization during lyophilization. Polysorbate 20 acts as a stabilizer in injections of biologics, and is capable of effectively reducing protein aggregation and enhancing long-term storage stability of a medicament. Arginine functions as a protective agent, and is capable of effectively suppressing protein aggregation. Because the formulation needs to maintain a physiological osmolality range, while meeting the requirements on the excipient concentration by lyophilized formulations, examined concentration ranges of 5%-9% trehalose and 1%-5% arginine were selected. Further, most of the pharmaceutical formulations comprise polysorbate 20 in a concentration of 0.0003%-0.3% (w/v) (if any), so by referring to marketed antibody formulations, an examined concentration range of 0.01%-0.03% polysorbate 20 was selected.

The formulations examined are shown in Table 6.

**Table 6. Experiment design of auxiliary materials screening**

| Number of the formulation | Buffer | ADC concentration (mg/mL) | Arginine concentration (%) | Trehalose concentration (%) | pH | Polysorbate 20 concentration (%) |
|---|---|---|---|---|---|---|
| A | | 10 | 1 | 5 | 7.4 | |
| B | | 10 | 1 | 7 | 7.4 | |
| C | | 10 | 1 | 9 | 7.4 | |
| D | | 10 | 3 | 7 | 7.4 | |
| E | 10 mmol/L PB | 15 | 3 | 7 | 7.4 | |
| F | | 20 | 3 | 7 | 7.4 | |
| G | | 10 | 1 | 7 | 7.4 | |
| H | | 10 | 3 | 7 | 7.4 | |
| I | | 10 | 5 | 7 | 7.4 | |
| J | | 10 | 3 | 7 | 7.4 | 0.01 |
| K | | 10 | 3 | 7 | 7.4 | 0.03 |

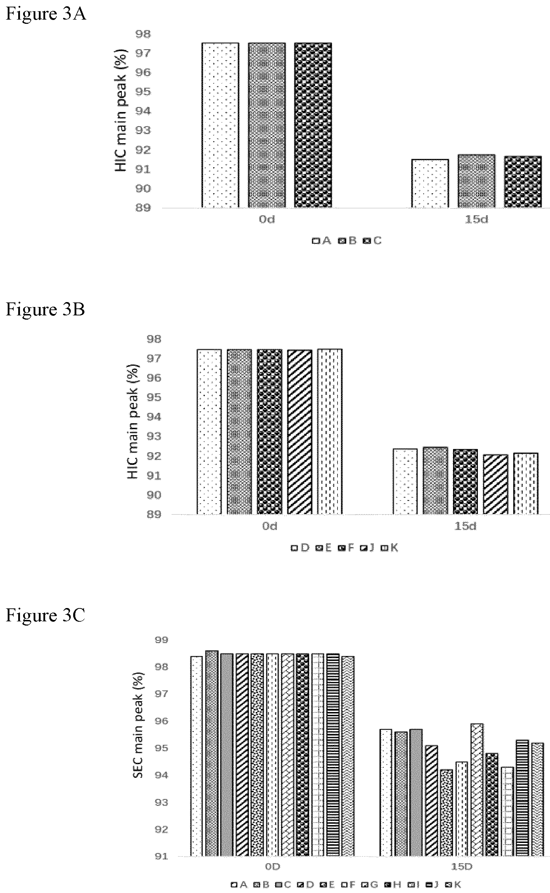

The experiment results demonstrated that: in the accelerated process, 1) When an amount of 5-9% trehalose was comprised, the purity by SEC and the shedding rates of the small molecule drugs exhibited minimal difference (shown in Table 7 & Figure 3A), then a concentration of 7% trehalose was selected to meet osmolality requirements while adhering to the minimal auxiliary material principle; 2) The shedding rates of the small molecule drugs enhanced with increasing arginine concentrations, and the results by SEC showed arginine had a small influence on the generation of aggregates (shown in Table 7 & Figure 3C), arginine therefore was excluded to adhere to the minimal auxiliary material principle also; 3) With increasing ADC concentrations, the shedding rates of the small molecule drugs enhanced and the purity by SEC showed a decreasing tendency (shown in Table 7 & Figure 3B and Figure 3C), therefore a concentration of 10 mg/mL was selected for the ADC; 4) When an amount of 0.01-0.03% polysorbate 20 was comprised, the purity by SEC and the shedding rates of the small molecule drugs showed no obvious difference (shown in Table 7 & Figure 3B and Figure 3C), then a moderate concentration of 0.02% polysorbate 20 was selected.

Based on a comprehensive analysis on the results above, a preferred composition of a formulation was finally established: 10 mg/mL ADC, 10 mmol/L PB, 7% trehalose, 0.02% polysorbate 20, pH 7.4. The formulation exhibited an osmolality of 200-300 mOsmol/kg, as detected. In view that the present product is to be intravenously dripped through an isotonic compatible solution, such a composition will not drastically affect the osmotic pressure at the time of administration, and so the composition of the formulation established is scientifically feasible.

**Table 7. Experiment results of auxiliary materials screening**

| Number of the formulation | Arginine concentration (%) | Trehalose concentration (%) | ADC concentration (mg/mL) | Polysorbate 20 concentration (%) | Purity by SEC (%) | | | Shedding rate of the small molecule drugs (%) | | |
|---|---|---|---|---|---|---|---|---|---|---|
| | | | | | 0 d | 15 d | 30 d | 0 d | 15 d | 30 d |
| A | 1 | 5 | 10 | | 98.4 | 95.7 | 93.2 | ND | 0.40 | 2.03 |
| B | 1 | 7 | 10 | | 98.6 | 95.6 | 93.7 | ND | 0.34 | 1.76 |
| C | 1 | 9 | 10 | | 98.5 | 95.7 | 93.6 | ND | 0.69 | 2.07 |
| D | 3 | 7 | 10 | | 98.5 | 95.1 | 92.2 | ND | 0.63 | 1.93 |
| E | 3 | 7 | 15 | | 98.5 | 94.2 | 92.0 | ND | 0.77 | 2.41 |
| F | 3 | 7 | 20 | | 98.5 | 94.5 | 91.8 | ND | 0.85 | 2.17 |
| G | 1 | 7 | 10 | | 98.5 | 95.9 | 94.0 | ND | 0.69 | 1.71 |
| H | 3 | 7 | 10 | | 98.5 | 94.8 | 91.5 | ND | 0.50 | 1.40 |
| I | 5 | 7 | 10 | | 98.5 | 94.3 | 91.8 | ND | 0.72 | 2.33 |
| J | 3 | 7 | 10 | 0.01 | 98.5 | 95.3 | 92.1 | ND | 1.33 | 2.87 |
| K | 3 | 7 | 10 | 0.03 | 98.4 | 95.2 | 93.3 | ND | *1.55* | 3.23 |

| | | | | | | | | | | |
|---|---|---|---|---|---|---|---|---|---|---|
| Note: "ND" means no small molecule drug shedding was detected. | | | | | | | | | | |

A stock solution of process validation batch was prepared following the composition of the formulation (10 mg/mL ADC, 10 mmol/L PB, 7% trehalose, 0.02% polysorbate 20, pH 7.4), and the long-term stability of the stock solution was studied at the storage temperature (≤ -60 °C).

The results provided in Table 8 showed, the stock solution having the composition maintained good stability even after stored for 24 months. Therefore, the formulation composition is stable and feasible.

**Table 8. Experiment results of long-term stability of a stock solution of process validation batch**

| Study period (month) | Main peak (SEC) (%) | Main peak (HIC) * (%) |
|---|---|---|
| 0 | 98.7 | 96.9 |
| 3 | 99.2 | 97.4 |
| 6 | 99.0 | 97.1 |
| 9 | 99.2 | 97.0 |
| 12 | 99.1 | 97.2 |
| 18 | 99.1 | 97.2 |
| 24 | 99.3 | 97.2 |

| | | |
|---|---|---|
| *: profiling the small molecule drug shedding (the higher the main peaks, the better the stability of the small molecule drugs). | | |

### Example 5 Establishment of lyophilization process and preparation of lyophilized formulation

### I. Development of lyophilization process parameters

### a) Determination of key temperatures

Several key temperatures (eutectic temperature, glass transition temperature, and collapse temperature) in a lyophilization process significantly impact product quality, so these key temperatures should be determined prior to establishing the lyophilization process.

Eutectic temperature (Te): when a certain temperature is reached, the composition of the liquid phase becomes totally identical to that of the formed solid phase in a mixed solution, and the temperature at this time is eutectic temperature of the solution.

Glass transition temperature (Tg): during freezing, as ice crystals form, the remaining solution becomes increasingly concentrated, and when a certain concentration is reached, the residual moisture no longer crystallizes and at this moment, the solution reaches the temperature corresponding to the maximum freezing concentration state.

Collapse temperature (Tc): during lyophilization, when the temperature of the dried layer rises to a certain point, the ice in the substance has sublimated, and the dried layer exhibits a porous honeycomb and spongy structure, which is temperature-dependent. When the temperature of the solid matrix in the structure rises while the rigidity of the solid matrix decreases below a threshold value, closure of vapor diffusion channels will be caused, and the temperature at this moment is called as collapse temperature.

The three key temperatures in lyophilization were measured using freeze-drying microscopy, cryogenic differential scanning calorimetry (DSC) and eutectic point probes. The three temperatures of the liquid formulation established in Example 4 were determined to be: eutectic temperature: -18.3 °C; glass transition temperature: -31.2 °C; and collapse temperature: -27.2 °C. The results are provided in Table 9.

**Table 9. Measurements of the key temperatures**

| Sample name | Collapse temperature | Glass transition temperature | Eutectic temperature |
|---|---|---|---|
| Liquid formulation * | -27.2 °C | -31.2 °C | -18.3 °C |

| | | | |
|---|---|---|---|
| *: the composition of the formulation is: 10 mg/mL ADC, 10 mmol/L PB, 7% trehalose, 0.02% polysorbate 20, pH 7.4. | | | |

### b) Determination of pre-freezing conditions

Pre-freezing rate, pre-freezing temperature, and pre-freezing time in a pre-freezing process need to be considered. The pre-freezing rate has an influence on crystal size, sublimation rate and protein activity. For example, a slow freezing rate leads to large crystals, high sublimation rate, and less damaged protein. A slow freezing mode, i.e., cooling the samples together with the shelves of the freezing dryer, was adopted for the liquid formulation provided in the present disclosure. Generally, the pre-freezing temperature is 20 °C lower than the eutectic temperature. The eutectic temperature of the formulation was detected to be -18.3 °C, and in consideration of the influence of the performance of different freeze dryers and radiation heat in the process, the temperature of the shelves for pre-freezing was set to ≤ -40 °C and the pre-freezing time was set to at least 2 hours.

Annealing is a process of raising the temperature of a frozen product to a temperature lower than the eutectic temperature at a certain heating rate, keeping that temperature for a period of time, and then lowering the temperature to the pre-freezing temperature at a certain cooling rate. Introducing an annealing procedure to the pre-freezing process can strengthen crystallization and remove crystal components with lower Tg values, thereby improving the overall Tg and shortening the primary drying time. The formulation provided in the present disclosure comprises trehalose, which has a low collapse temperature, and requires a long primary drying time. Therefore, an annealing procedure was added into the pre-freezing process. Further, the annealing temperature is generally above the glass transition temperature and below the eutectic temperature. The glass transition temperature of the formulation was detected to be -31.2 °C, and in consideration of the influence of the difference between the temperature of the shelves and the actual temperatures of the samples and radiation heat in the pre-freezing process, the temperature of the shelves for annealing were set to -35 °C - -20°C which would be kept for at least 2 hours; and then the temperature of the shelves was lowered to ≤ -40 °C which would be kept for at least 2 hours.

### c) Determination of sublimation conditions

The sublimation process included two stages: primary drying and secondary drying.

The primary drying stage requires the control of temperature and pressure. The primary drying stage removes most of the free moisture in a sample, so rate of the primary drying will influence the time of a freeze-drying process. The rate of the primary drying can be improved by increasing the freeze-drying temperature and pressure. In a freeze-drying process, the actual temperature of a product needs to be ensured to be lower than the collapse temperature of the product; sublimation is an endothermic process, so the temperature of the product needs to be lower than the temperature of the shelves at the initial phase of the sublimation; and the sublimation rate can be improved by increasing the temperature of the shelves, therefore the temperature of the shelves needs to be set higher than the collapse temperature of the product, and the temperature of the actual sublimation interface needs to be lower than the collapse temperature of the product. The collapse temperature of the formulation was detected to be -27.2 °C, and in consideration of the influence of the performance of different freeze dryers and radiation heat in the primary drying stage, the temperature of the shelves for primary drying were set to -20 ± 10 °C. In addition, the primary drying generally needs to be conducted under a pressure lower than half of the saturated vapor pressure of the product corresponding to the highest temperature of the product. Therefore, the pressure for primary drying was set to ≤ 0.2 mbar. Further, it was determined the primary drying stage should last at least 24 hours through a pressure rise testing of the primary drying stage.

The secondary drying is to heat the product at a relatively high temperature so as to further reduce the moisture content of the freeze-dried product. The secondary drying needs to be performed below the denaturation temperature of the product. Therefore, the temperature of the shelves for secondary drying was set to 20 ± 5 °C and the actual temperature of the samples would be lightly lower than that temperature of the shelves; and the secondary drying stage was determined to last at least 10 hours. The vacuum degree in the secondary drying stage has a minor influence on the product quality, so any proper vacuum degree would be okay for the secondary drying stage.

### II. Validation of the lyophilization process

As above, lyophilization process parameters were established. The parameters are provided in Table 10.

**Table 10. Lyophilization process parameters**

| Step | Parameters (shelf temperature and pressure) | Duration time |
|---|---|---|
| Pre-freezing | ≤ **-**40 °C | ≥ 2 h |
| Annealing | -35 °C - -20 °C | ≥ 2 h |
| Pre-freezing | ≤ **-**40 °C | ≥ 2 h |
| Primary drying (sublimation drying) | -20±10 °C; and pressure ≤ 0.2 mbar | ≥ 24 h |
| Secondary drying (desorption drying) | 20±5 °C; and pressure ≤ 0.2 mbar | ≥ 10 h |

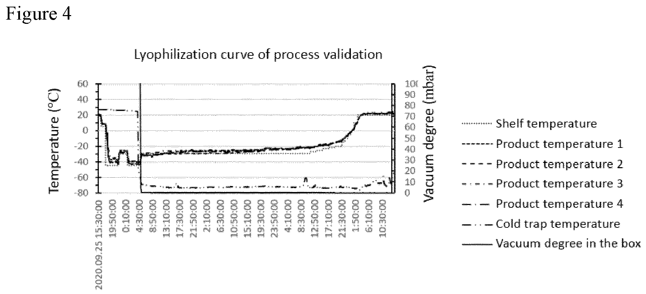

The liquid formulation established in Example 4 was lyophilized using the lyophilization process as follows to validate the lyophilization process. The lyophilization curve is shown in Figure 4.

Pre-freezing process: the temperature of a sample was lowered to ≤ -40 °C and kept for at least 2 hours; then the temperature of the sample was raised to a temperature above the glass transition temperature but below the eutectic temperature (-35 °C to -20 °C) and kept for at least 2 hours, allowing the sample to recrystallize (this step can shorten the time required for the primary drying); and after the annealing, the temperature of the sample was further lowered to ≤ -40 °C and kept for at least 2 hours, allowing the sample to maintain its rigidity structure further.

Primary drying stage: when the pre-freezing process finished, the vacuum degree of the freeze-dryer chamber was controlled to a pressure of ≤ 0.2 mbar, and the temperature of the sample was raised to -20 ± 10 °C and kept for at least 24 hours. The primary drying was stopped when the freeze-dryer chamber passed the pressure rise testing (no fixed standard, varied between different freeze-dryers).

Secondary drying stage: when the primary drying stage finished, the temperature of the sample was raised to 20 ± 5 °C and kept for at least 10 hours. Then the secondary drying stage ended.

The experiment results showed the lyophilized products of three lyophilized batches all demonstrated a moisture content of less than 3%, good appearance, and rapid reconstitution in water for injection. No foreign matters and very few insoluble particulates were observed in the liquid formulations obtained after the reconstitution; and as determined by detections, key quality attributes of the products did not obviously change, and the consistency was good, indicating the feasibility of the lyophilization process. The results are provided in Table 11.

**Table 11. Results of the validation of the lyophilization process**

| Detection item | Batches | | |
|---|---|---|---|
| | Process validation batch 1 | Process validation batch 2 | Process validation batch 3 |
| Moisture (%) | 0.9 | 0.9 | 0.9 |
| Appearance | White or yellowish loose body, without signs of thawing | White or yellowish loose body, without signs of thawing | White or yellowish loose body, without signs of thawing |
| Reconstitution time | Dissolved completely in 15 minutes | Dissolved completely in 15 minutes | Dissolved completely in 15 minutes |
| Visible foreign matters | None | None | None |
| Insoluble particulates | ≥ 10 µm: 7; | ≥ 10 µm: 16; | ≥ 10 µm: 21; |
| | ≥ 25 µm: 1 | ≥ 25 µm: 1 | ≥ 25 µm: 1 |
| pH | 7.4 | 7.5 | 7.5 |
| Osmolality | 261 mOsmol/kg | 242 mOsmol/kg | 241 mOsmol/kg |
| SEC (%) | 99.0 | 98.9 | 99.0 |
| Contents of free small molecule drugs | MMAE: < 0.3%; | MMAE:< 0.31%; | MMAE: < 0.33%; |
| | L20E: < 0.08%; | L20E: < 0.07%; | L20E: 0.08%; |
| | BL20E: < 0.07%; | BL20E: < 0.06%; | BL20E: < 0.06%; |
| | In total: < 0.45% | In total: < 0.44% | In total: < 0.47% |

The above description of the embodiments of the invention is not intended to limit the invention, and those skilled in the art may make various changes and modifications to the present invention without departing from the spirit of the invention, which should fall within the scope of the appended claims.

## Claims

1. A liquid formulation comprising an anti-Nectin-4 antibody-drug conjugate or salt thereof as well as a buffer and an auxiliary material; **characterized in that**, the anti-Nectin-4 antibody-drug conjugate or salt thereof has a formula: Ab-[L-CTD]m, in which
Ab represents an anti-Nectin-4 antibody or fragment thereof comprising three heavy chain complementarity determining regions, i.e., H-CDR1, H-CDR2, and H-CDR3, and three light chain complementarity determining regions, i.e., L-CDR1, L-CDR2, and L-CDR3, wherein the H-CDR1 comprises the amino acid sequence (DYGVS) shown in SEQ ID NO. 3, the H-CDR2 comprises the amino acid sequence (VIWGGGKIYYNSVLKS) shown in SEQ ID NO. 4, and the H-CDR3 comprises the amino acid sequence (QGGLLFYAMDY) shown in SEQ ID NO. 5; and, the L-CDR1 comprises the amino acid sequence (KSSQSLLNTYSQKNYLA) shown in SEQ ID NO. 8, the L-CDR2 comprises the amino acid sequence (FASTRES) shown in SEQ ID NO. 9, and the L-CDR3 comprises the amino acid sequence (QQHYNTPFT) shown in SEQ ID NO. 10;
L represents a linker;
CTD represents a drug; and
m represents the average number of drug molecules conjugated to one Ab molecule (average DAR).

2. The liquid formulation according to claim 1, **characterized in that**, in the anti-Nectin-4 antibody-drug conjugate or salt thereof, CTD is a cytotoxic drug; preferably, CTD is one or more selected from the group consisting of: microtubule inhibitors MMAE, DM1, DM4, Tublysin, amanitin, cachimycin, Eribulin and derivatives thereof; topoisomerase inhibitors SN38, Exatecan and derivatives thereof; and DNA binding agents PBD, doxorubicin and derivatives thereof;
m is 1.0 to 5.0, preferably 3.0 to 4.2, more preferably 3.5 to 4.5, still more preferably 3.8 to 4.2, yet more preferably 3.9 to 4.1, and particularly preferably 4.0.

3. The liquid formulation according to claim 1 or 2, **characterized in that**, the anti-Nectin-4 antibody-drug conjugate or salt thereof has a structure represented by the formula I as follows: 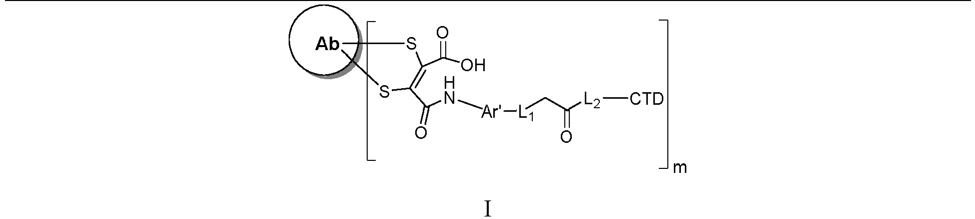 in which:
Ab is the anti-Nectin-4 antibody or fragment thereof;
Ar' is any one selected from the group consisting of: substituted or unsubstituted C6-C10 arylene and substituted or unsubstituted 5-12 membered heteroarylene, wherein the substitution refers to the replacement of a hydrogen atom on a group by one or more substituents selected from the group consisting of: halogen (F, Cl, Br or I), halogenated alkyl (e.g. halogenated C1-C6 alkyl, preferably halogenated C1-C4 alkyl, e.g. trifluoromethyl) and alkoxy (e.g. C1-C6 alkoxy, preferably C1-C4 alkoxy, e.g. methoxy);
L₁ is -O(CH2CH2O)n- linked to Ar', in which n is any integer in the range from 1 to 24, preferably from 1 to 10, more preferably from 3 to 5;
L₂ is an enzyme cleavable fragment, which is selected from the group consisting of a dipeptide or a tripeptide or a tetrapeptide or a combination of the dipeptide or the tripeptide or the tetrapeptide with a self-immolative linker, such as Val-Ala, Val-Ala-PAB, Val-Cit, Val-Cit-PAB, Phe-Lys-PAB, Ala-Ala-Ala, Gly-Gly-Phe-Gly (GGFG), MAC glucuronide phenol;
preferably, L₂-CTD is VcMMAE, GGFG-Dxd or VC-seco-DUBA;
preferably, in the case that Ar' is a substituted or unsubstituted 5-12 membered heteroarylene, the heteroatom is N;
preferably, Ar' is a substituted or unsubstituted C6 arylene or a substituted or unsubstituted 6 membered heteroarylene.

4. The liquid formulation according to any one of claims 1 to 3, **characterized in that**, the anti-Nectin-4 antibody-drug conjugate or salt thereof has a structure as follows:

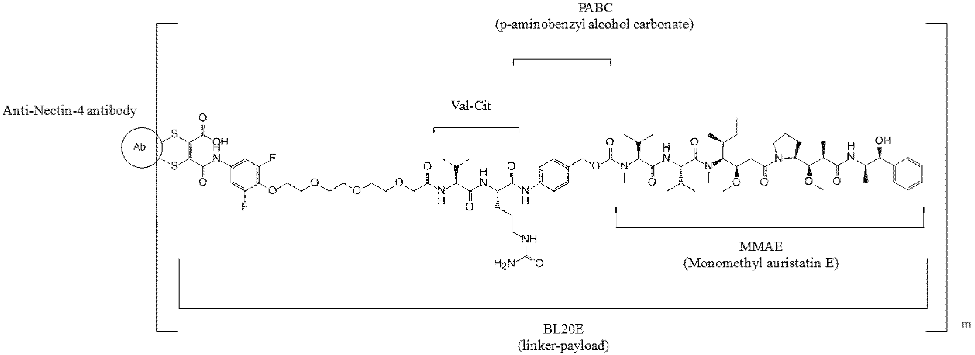

5. The liquid formulation according to any one of claims 1 to 4, **characterized in that**, the anti-Nectin-4 antibody or fragment thereof comprises a heavy chain variable region (VH) comprising the amino acid sequence shown in SEQ ID NO: 2 or a variant thereof; and, the anti-Nectin-4 antibody or fragment thereof comprises a light chain variable region (VL) comprising the amino acid sequence shown in SEQ ID NO: 7 or a variant thereof;
preferably, the anti-Nectin-4 antibody or fragment thereof comprises a heavy chain (HC) comprising the amino acid sequence shown in SEQ ID NO: 1 or a variant thereof; and, the anti-Nectin-4 antibody or fragment thereof comprises a light chain (LC) comprising the amino acid sequence shown in SEQ ID NO: 6 or a variant thereof.

6. The liquid formulation according to any one of claims 1 to 5, **characterized in that**, the liquid formulation is in the form of a solution, an emulsion or a suspension;
preferably, the liquid formulation is a liquid composition for parenteral administration;
preferably, the liquid formulation is a liquid composition for intravenous administration, such as for intravenous drip.

7. The liquid formulation according to any one of claims 1 to 6, **characterized in that**, in the liquid formulation, the buffer is one or more selected from the group consisting of phosphate buffer, acetate buffer, and Tris-HCl buffer; preferably, the phosphate buffer comprises a mixture of sodium dihydrogen phosphate and disodium hydrogen phosphate; preferably, the acetate buffer comprises a mixture of acetic acid and sodium acetate;
preferably, the liquid formulation further comprises an auxiliary material;
preferably, the auxiliary material comprises a sugar, preferably trehalose;
preferably, the auxiliary material comprises a surfactant, preferably polysorbate 20 or polysorbate 80;
preferably, the liquid formulation has a pH of 5.0-8.0, preferably 7.0-8.0, more preferably 7.0-7.8, such as 7.2, 7.4, 7.6, or 7.8.

8. The liquid formulation according to any one of claims 1 to 7, **characterized in that**, the liquid formulation comprises the anti-Nectin-4 antibody-drug conjugate or salt thereof in a concentration of 5-40 mg/mL, preferably 8-30 mg/mL, more preferably 10-20 mg/mL;
preferably, the liquid formulation comprises the phosphate buffer acting as a buffering system; preferably, the liquid formulation comprises the phosphate buffer in a concentration of 5-20 mmol/L, preferably 8-15 mmol/L, more preferably 8-10 mmol/L;
preferably, the liquid formulation comprises trehalose; preferably, the liquid formulation comprises trehalose in a concentration of 5%-9% (w/v), preferably 6%-8% (w/v), more preferably 7% (w/v);
preferably, the liquid formulation comprises polysorbate 20 or polysorbate 80, such as polysorbate 20 or polysorbate 80 in a concentration of 0.0003%-0.3% (w/v); preferably, the liquid formulation comprises polysorbate 20 in a concentration of 0.0003%-0.3% (w/v), preferably 0.01%-0.03% (w/v), more preferably 0.02%-0.03% (w/v).

9. The liquid formulation according to any one of claims 1 to 8, **characterized in that**, the liquid formulation comprises:
5-40 mg/mL the anti-Nectin-4 antibody-drug conjugate or salt thereof;
5-20 mmol/L the phosphate buffer;
5%-9% (w/v) the sugar, such as trehalose; as well as
0.0003%-0.3% (w/v) the surfactant, such as polysorbate 20;
and, the liquid formulation has a pH of 5.0-8.0;
or, the liquid formulation comprises:
8-30 mg/mL the anti-Nectin-4 antibody-drug conjugate or salt thereof;
5-20 mmol/L the phosphate buffer;
6%-9% (w/v) the sugar, such as trehalose; as well as
0.01%-0.03% (w/v) the surfactant, such as polysorbate 20;
and, the liquid formulation has a pH of 7.0-8.0, such as 7.2-7.8;
or, the liquid formulation comprises:
10-30 mg/mL the anti-Nectin-4 antibody-drug conjugate or salt thereof;
8-15 mmol/L the phosphate buffer;
6%-8% (w/v) the sugar, such as trehalose; as well as
0.02%-0.03% (w/v) the surfactant, such as polysorbate 20;
and, the liquid formulation has a pH of 7.2-7.8;
or, the liquid formulation comprises:
10-20 mg/mL the anti-Nectin-4 antibody-drug conjugate or salt thereof;
8-10 mmol/L the phosphate buffer;
6%-8% (w/v) the sugar, such as trehalose; as well as
0.02%-0.03% (w/v) the surfactant, such as polysorbate 20;
and, the liquid formulation has a pH of 7.2-7.6;
or, the liquid formulation comprises:
10 mg/mL the anti-Nectin-4 antibody-drug conjugate or salt thereof;
10 mmol/L the phosphate buffer;
7% trehalose; as well as
0.02% polysorbate 20;
and, the liquid formulation has a pH of 7.4.

10. A solid formulation obtained by solidifying the liquid formulation as defined in any one of claims 1 to 9;
preferably, the solid formulation is a lyophilized formulation, such as lyophilized powder injection.

11. The solid formulation according to claim 10, **characterized in that**, the solid formulation has a moisture content of no greater than 3.0%, preferably of no greater than 1.5%, more preferably of no greater than 1.0%.

12. A reconstituted formulation obtained by dissolving the solid formulation as defined in claim 10 **or 11** in a solvent.

13. Use of the formulation as defined in any one of claims 1 to 12 in the manufacture of a medicament for treating a tumor.

14. A method for treating a tumor, comprising administering to a subject in need thereof the formulation as defined in any one of claims 1 to 12, or a formulation obtained by further formulating the formulation as defined in any one of claims 1 to 12.

15. A kit, comprising the formulation as defined in any one of claims 1 to 12, or a formulation obtained by further formulating the formulation as defined in any one of claims 1 to 12.
